# EUROPEAN PATENT APPLICATION

(11) **EP 4 322 104 A1**
(43) Date of publication of application: **14.02.2024**
(21) Application number: 22189730.9
(22) Date of filing: 10.08.2022
(51) Int. Cl.: G06T 5/50, G06T 7/215

(54) **DATA PROCESSING DEVICE AND COMPUTER-IMPLEMENTED METHOD FOR MOTION-DEPENDENT IMAGE PROCESSING IN A MEDICAL OBSERVATION DEVICE AND MEDICAL OBSERVATION DEVICE**

(71) Applicant: Leica Instruments (Singapore) Pte Ltd, Singapore 608924 (SG)
(72) Inventor: Themelis, George, 88131 Lindau (DE); Honegger, Marc, 9320 Arbon (CH)
(74) Representative: Grünecker Patent- und Rechtsanwälte PartG mbB

(57) **Abstract**

The invention relates to a data processing device (100) for motion-dependent image processing in a medical observation device, such as a microscope or endoscope. In the art, low light imaging at video frame rates either has the drawback of images that are overly noisy or that show a motion blur. Image quality is improved by the data processing device (100) being configured to retrieve an input set (102a) of input images from a plurality of input images (102), the plurality of input images being part of one or more time sequences of input images, wherein the input images of the input set (102a) are all from the same time sequence of input images, and wherein each input image comprises input pixels, each input pixel comprising an intensity value; determine a stationary input image segment (104) in the input images of the input set (102a) by applying a motion detection scheme to the input set (102a) , the stationary input image segment (104) representing stationary image content in the input set (102a); determine a stationary output image segment (108) in an output image (106) , the stationary output image segment (108) having the same location in the output image (106) as the stationary input image segment (104) has in one of the input images of the input set (102a); and determine an intensity value of an output pixel of the output image (106) in the stationary output image segment (108) from a combination of intensity values combining corresponding input pixels from different input images of a subset of input images with each other, wherein the corresponding pixels lie within the stationary input image segment (104).

## Description

The invention relates to a data processing device for motion-dependent image processing in a medical observation device such as a microscope or endoscope, to a medical observation device, such as a microscope or endoscope, to a computer-implemented method for motion-dependent image processing of medical upset relational input images, and to a corresponding computer-readable medium or computer program product.

In applications of low light imaging such as fluorescence and surgical microscopy, the sensitivity of an image acquisition device, e.g. of a camera may be seen as a bottleneck in the imaging process. In low light imaging scenarios, a fluorescence intensity may be too weak to provide a clear picture of the imaged scene, in particular if images are captured at video frame rates. If camera gain is increased image noise decreases image quality.

If an observed object is static, i.e. not moving, one possible approach to improve image quality is the application of an increased integration time at the expense of a reduced frame rate. Even if this approach can offer substantial improvement in image quality of static objects, it suffers from motion blur when the object is moving.

In the art, low light imaging at video frame rates therefore either has the drawback of images that are overly noisy or that show a motion blur.

One object of the present invention is therefore to improve the efficiency of imaging processing, e.g. by offering a substantially better image quality with a given image acquisition device, or by offering the same image quality with a less sensitive and/or cheaper and/or smaller image acquisition device.

This object is solved for the data processing device mentioned in the beginning by the invention in that the data processing device is configured to retrieve an input set of input images from a plurality of input images, the plurality of input images being part of one or more time sequences of input images, wherein the input images of the input set are all from the same time sequence of input images, and wherein each input image comprises input pixels, each input pixel comprising an intensity value, to determine a stationary input image segment in the input images of the input set by applying a motion detection scheme to the input set, the stationary input image segment representing stationary image content in the input set, to determine a stationary output image segment in an output image, the stationary output image segment having the same location in the output image as the stationary input image segment has in one of the input images of the input set, and to determine an intensity value of an output pixel of the output image in the stationary output image segment from a combination of intensity values combining corresponding input pixels from different input images of a subset of input images with each other, wherein the corresponding pixels lie within the stationary input image segment.

This has the advantage that the imaging process may be improved because the camera settings may be adjusted dynamically in real time.

The medical observation device mentioned in the beginning achieves the above object by comprising a data processing device according to the invention and an input camera for recording a time sequence of the one or more time sequences. The medical observation device also has the advantage of an improved imaging process. The medical observation device may, for example, be a microscope or an endoscope.

The computer-implemented method for motion-dependent image processing of medical observational input images mentioned in the beginning solves the above problem by comprising the method steps of retrieving an input set of input images from a plurality of input images, the plurality of input images being part of one or more time sequences of input images, wherein the input images of the input set are all from the same time sequence of input images, and wherein each input image comprises input pixels, each input pixel comprising an intensity value, of determining a stationary input image segment in the input images of the input set by applying a motion detection scheme to the input set, the stationary input image segment representing stationary image content in the input set, of determining a stationary output image segment in an output image, the stationary output image segment having the same location in the output image as the stationary input image segment has in one of the input images of the input set, and of determining an intensity value of an output pixel in the stationary output image segment of the output image from a combination of intensity values combining corresponding input pixels from different input images of a subset of input images with each other, wherein the corresponding pixels lie within the stationary input image segment.

The inventive computer-implemented method also has the advantage of improved image processing.

The inventive computer-readable medium or computer program product solves the above problem by comprising instructions which, when the program is executed by a computer, cause the computer to carry out the computer-implemented method according to the invention.

In the following, further improvements of the inventive data processing device, the inventive medical observation device, the inventive computer-implemented method and the computer-readable medium or computer program product will be described. Each of the described embodiments is advantageous on its own, and additional features may be arbitrarily combined with one another or omitted.

The improvements described with respect to the data processing device or the medical observation device and their advantages also relate to the computer-implemented method or the computer-readable medium or computer program product. Likewise, the improvements and advantages mentioned with respect to the method for the computer-readable medium or computer program product also apply to the data processing device or the medical observation device, according to the invention. Features described with respect to the data processing device also relate to the medical observation device.

The stationary input image segment may correspond to the entire input image. In other words, the entire input image of the input set of input images from the plurality of input images is stationary.

Accordingly the stationary output image segment in the output image also corresponds to the entire output image. In this embodiment, every pixel of the output image is thus equally processed, i.e. by determining an intensity value of every output pixel in the stationary output image from a combination of intensity values combining corresponding input pixels from different input images of a subset of input images with each other, wherein the corresponding pixels lie within the stationary input images.

The intensity value of an output pixel of the output image may, in this case, be determined from a combination of intensity values combining corresponding input pixels from different input images of a subset of input images with each other, wherein the corresponding pixels lie within the input image that corresponds to the stationary input image segment.

The stationary output image segment may have the same location in the output image as the stationary input image segment has in the most recent of the input images of the input set.

A stationary segment may in particular be defined by pixels of which the variation of the intensity values across a predetermined number of input images is below a predetermined variation threshold.

A stationary input image segment may be determined for each input image of the input set. In a particular embodiment, each stationary input image segment may correspond to the entire corresponding input image of the input set.

The stationary input image segment may comprise contiguous and/or non-contiguous clusters of pixels. In other words, the stationary input image segment may comprise one cluster of adjacent pixels or more than one of such clusters of adjacent pixels. The stationary input image segment may thus be separated from one another by non-stationary input image segments.

The stationary input image segment may in particular have the same location, shape, orientation and/or size in each of the input images of the input set.

The stationary input image segment may be representable of an image pattern. The same image pattern may be processed in each of the input images. Such an image pattern may be determined by a pattern recognition scheme, such as k-means clustering, machine learning, object recognition or other known processes.

In one embodiment, the image pattern may be at different locations in at least some of the input images. This may, for instance, be the case if a sample is slowly scanned, wherein the image pattern may be moved with the scan speed. Also a relative movement may be seen in the field of view. Such an image pattern is representative for a stationary input image segment.

In order to prevent any wrong determination of stationary image content, each of the stationary image content may represent image content of which motion is below a predetermined motion threshold. Vibrations that may occur in the imaging process therefore do not falsify the determination of stationary input image segments.

The above-mentioned motion detection scheme may be any of a known motion detection scheme or motion segmentation scheme such as moving object detection, frame differencing, temporal differencing or similar schemes.

A corresponding pixel is a pixel at the same location within the stationary input image segment. It is advantageous if the output pixels of the output image are obtained pixel-wise. In other embodiments clusters of output pixels of the output image are obtained from clusters of pixels of the input images.

In some embodiments more than one time sequence of input images may be present. Each of the two or more time sequences may be generated by a different camera. In other embodiments some of the time sequences may be generated by a first camera and another time sequence may be generated by a second camera. A camera is representative of any possible image acquisition device.

A corresponding embodiment of the inventive medical observation device therefore further comprises a second camera for recording a second time sequence of the one or more time sequences. The second camera may generate the second input images.

In one advantageous embodiment of the medical observation device, the input camera may be a white light camera and/or the second camera may be a fluorescence light camera configured to record an emission spectrum of at least one fluorophore.

This has the advantage that information may be retrieved from different spectral regions revealing different information. Those information may be efficiently combined with one another.

The data processing device may provide a threshold parameter that is representative of the size of the subset. This threshold parameter may be provided as a default value or may be changed by the user of the data processing device. The threshold parameter may be received by the data processing device and, in another embodiment, may be stored in a storage unit. This has the advantage that single pixels or clusters of two, three, four, or general: few pixels that show some noise in the input image data are determined to be non-stationary. Any number of pixels may be covered by the threshold parameter. This allows the suppression of the influence of pixel noise for improving image quality.

In another advantageous embodiment, the data processing device is further configured to determine an intensity value of an output pixel of the output image outside from the stationary output image segment from one or more corresponding input pixels of the subset of the input images outside the stationary input image segment, wherein a number of input pixels used for determining the intensity value of the output pixel outside the stationary output image segment being smaller than a number of input pixels used for determining the intensity value of an output pixel in the stationary output image segment. Outside the stationary input image segment, a movable object may be detected. In the sequence of plurality of input images of the input set, this moved object will be located at different positions in the individual input images. Different input pixels of individual input images are used for determination of the intensity value of the output pixel outside the stationary output image segment. The different input pixels of adjacent Input images may describe the trajectory of the moving element that is represented by the output pixel in the output image. Consequently, a multitude of input pixels may determine the intensity value of one output pixel. The number of input pixels is therefore higher than the number of output pixels.

In a further advantageous embodiment of the data processing device, the data processing device is further configured to determine the intensity value of an output pixel outside the stationary output image segment as the intensity value of a corresponding input pixel of the most recent input image of the subset of the input images. This has the advantage that no image blur may occur. The intensity value of the non-stationary image content is thus not calculated from a multitude of input images, but only from the most recent input image.

The data processing device may be further improved in that the number of input pixels used for determining the intensity value of an output pixel in the stationary output image segment is in the range of 3 to 9. The number of input pixels Used may thus be three, four, five, six, seven, eight or nine. In other embodiments, other numbers of input pixels is conceivable, e.g. one or two, or 10 or more. It may be possible to change said number of input pixels in dependence on the intensity value of the input pixels. By changing the number of input pixels for determining the intensity value of the output pixel, the intensity value of the stationary output image segment may be increased or decreased.

The data processing device may be further configured to retrieve a second set of second input images from the plurality of input images, wherein the second set of second input images are all from the same time sequence, the time sequence of the second set of input images being different from the time sequence of the input set, and wherein each second input image comprises second input pixels, each second input pixel comprising an intensity value. Such a data processing device may be further configured to determine a second stationary output image segment in each of the second input images of the second set, the second stationary output image segment being in a second output image and having the same location in the second output image as the stationary input image segment has in one of the input images of the input set, and to determine an intensity value of an output pixel in the second stationary output image segment of the second output image from a combination of the intensity values combining corresponding input pixels from different input images of a subset of the second set, wherein the corresponding pixels lie within the stationary input image segment.

Also in the case of a second set of the second input images, the second stationary output image segment may correspond to the entire second input image, i.e. the entire image content of the second input image is determined to be stationary.

The second output image may be computed from the subset of the second set of second input images, in particular, a size of the subset may be controlled by a user-controllable parameter. Such a user-controllable parameter may be input by a user and stored in a memory unit.

The input images of the input set and the second input images of the second set may be registered images. At least some images of the input set may have been recorded simultaneously with or as the second input images of the second set.

A further advantageous embodiment of the data processing device is further configured to register the input images of the input set and the second input images of the second set. If the different input images are registered, they may be distinguished from one another.

Further, the input images and second input images may be recorded simultaneously and/or synchronously. The input images and second input images may be recorded at identical times or within a time span that is preferably smaller than the flicker fusion threshold or less than 50 ms.

The data processing device may be further improved by being further configured to determine a size of the stationary input image segment, and to determine the intensity value of the output pixel of the output image in the stationary output image segment from the combination, when the determined size of the stationary input image segment exceeds a combination threshold.

This has the advantage that image content smaller than the combination threshold, i.e. image content extending over less than a certain number of pixels, is not processed by the data processing device. This may result in faster image processing. The size of the stationary input image segment may therefore comprise a count of pixels.

In another advantageous embodiment, the data processing device is further configured to determine a standard deviation for the output pixel in the stationary output image segment from the corresponding pixels of the different input images that lie within the stationary input image segment. This has the advantage that it is possible to reject an intensity value from a result of the combination combining the corresponding pixels, when the intensity value lies outside a threshold value based on the determined standard deviation.

The data processing device may be further configured to compute the output image, the stationary input image segment, or the stationary output image segment, and to provide the output image to a display device. Alternatively or additionally, the output image may be provided to a recording device. This recording device may in particular be a digital recording device that allows for further processing of the recorded image, e.g. saving into a memory.

In the following, the invention will be described by reference to the accompanying figures. The embodiments shown are exemplary and do not limit the present invention. Features shown in the different embodiments may be arbitrarily combined with one another or omitted. Further, features described with reference to a data processing device may be applied to a medical observation device as well as to a computer-implemented method. Accordingly, features described with reference to the method may be transferred to the corresponding apparatuses.

The figures show:
Fig. 1 a schematic view of a medical observation device;
Fig. 2 a flow diagram of a computer-implemented method;
Fig. 3 a flow diagram of a method for evaluating the input image sequence;
Fig. 4a and 4b flow diagrams for determining the stationary image region;
Fig. 5 a flow chart of a method to determine the stationary image region; and
Fig.6 another schematic flow chart for determining a stationary image region.

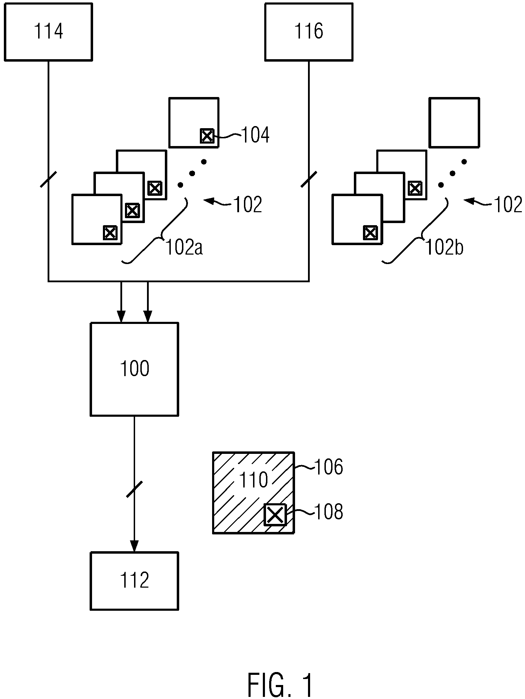

First, an example of an embodiment of the invention is described with reference to Fig. 1.

Fig. 1 schematically illustrates a medical observation device 100. The medical observation device 100 may be a fluorescence-based optical imaging device. Further, the medical observation device 100 may be embodied as a microscope or an endoscope. Although the medical observation device of Fig. 1 is only schematically illustrated as a microscope, the following description also applies to an endoscope.

The medical observation device 100 may be used to assist in surgery or in a laboratory. A user may observe an object or specimen 110 in an object scene 120 by using the medical observation device 100.

The medical observation device 100 may comprise a camera 130. The camera 130 may record an image of the object scene. The camera 130 may also be used as a video camera that records and provides an image sequence 132. A data processing device 140 may retrieve the image sequence as an input image sequence 132 from the camera. The data processing device 140 may be integrated into the medical observation device 100. Altematively, the data processing device 140 may be coupled to the medical observation device 100.

The medical observation device 100 may optionally comprise a secondary camera 150 in addition to the (primary) camera 130. The secondary camera may simultaneously observe the object scene 120 to produce another image sequence 152. The secondary camera 150 may thus also be embodied as a video camera which records the object scene 120 and provides the image sequence 152 to the data processing device 140. Any or all of the cameras 130 and 150 are configured to produce an image sequence of chronologically ordered images representing the object scene 120 observed by the medical observation device 100. Thus, according an embodiment where only one camera is present in the medical observation device 100, the data processing device 140 retrieves an input image sequence 132 of chronologically ordered primary input images.

According to another embodiment, when two cameras, such as the primary camera 130 and the secondary camera 150, observe the object scene 120 simultaneously, the data processing device 140 is configured to retrieve a primary input image sequence 132 of chronologically ordered primary input images representing the object scene 120 and a secondary input image sequence 152 of chronologically ordered secondary input images representing the object scene 120. As indicated above, the camera 130 may be embodied as a fluorescence camera. Alternatively it may be embodied as a white light camera. According to the embodiment comprising two cameras, the primary camera may be embodied as a white light camera and the secondary camera may be embodied as a fluorescence camera, the difference between a fluorescence camera and a white light camera being primarily that the fluorescence camera is configured to observe the object scene 120 through a passband filter (not shown in Fig. 1) which is also integrated in the camera, or part of the medical observation device 100. The passband filter may be selective to an emission light spectrum of a fluorophore that is present or added to the specimen 110 in the object scene 120.

As the passband filter may block any light component outside of the passband spectrum, the images captured by the fluorescence camera suffer from low light levels. This disadvantage is amplified when the camera operates with a very short exposure time. This is the case for any camera which operates in a video mode that produces an image sequence of chronologically ordered images. Independent of whether the camera is a white light camera or a fluorescence camera, or any camera of the medical observation device 100, a frame rate setting an exposure time setting may be dynamically adjusted by the data processing device 140. For example, the data processing device 140 may increase the frame rate of camera 130 and/or secondary camera 150. Increasing the frame rate may be useful to eliminate motion blur in single images of the image sequence that may arise, when a specimen 110 moves faster than the exposure time of the observing camera.

Furthermore, the primary camera 130 and secondary camera 150 of the medical observation device 100 may be arranged or adjusted or calibrated to produce images that can be overlaid. In other words, an image of the primary input image sequence from the first camera may be overlaid with a secondary image of the secondary input image sequence. In this case, the primary input images of the primary camera 130 and the secondary input images of the secondary camera 150 may have the same aspect ratio, the same pixel dimensions, the same image size and ratio, and/or have a predetermined known transformation relationship that allows to map input pixel of an image from the first camera to other input pixel of an image of the second camera. In other words, when the primary input image of the first camera comprises primary input pixel, the known relationship for the mapping allows to map the primary input pixel to a secondary input pixel of the secondary input image of the secondary camera.

The medical observation device may further comprise an output device 160. The output device 160 may receive an output image 170 from the data processing device 140. The output device 160 may be a display, smart glasses, a projector, a holographic display, or the like.

The output image 170 comprises output pixels, and each of the pixels comprises at least one intensity value. The data processing device 140 is configured to generate and/or output the output image 170.

According to an embodiment, the data processing device 140 may retrieve an input image sequence 132 from the camera 130. The input image sequence may comprise chronologically ordered input images representing the object scene 120 observed by the medical observation device 100. The data processing device 140 may then process the retrieved input image sequence 132 in order to output the output image 170. In particular, the data processing device 140 may evaluate the image sequence 132 to determine a motion in the object scene 120. When the image sequence 132 is not indicative of any motion being present in the observed object scene 120, the data processing device 140 may digitally enhance the most recent image of the image sequence 132 to provide an improved output image 170 to the output device 160. For example, the data processing device 140 may stack the most recent image of the retrieved image sequence 132 with antecedent images in the image sequence. For example, multiple images of the input image sequence 132 may be averaged to calculate the output image 170. Alternatively, the output image 170 may be calculated as a median filtered image from the input image sequence 132. The output image 170 may also be calculated by signal clipping from the retrieved input image sequence 132.

According to another embodiment, when instead of only one camera two cameras, that is, the first camera 130 and the secondary camera 150 are simultaneously observing the object scene 120, the data processing device 140 may analyze the primary input image sequence 132 to determine whether motion is present in the simultaneously observed object scene 120. In case the data processing device determines that there is no motion present in the observed object scene 120, the data processing device 140 may alternatively calculate the output image 170 from the secondary input image sequence, in the same manner as it is calculated when only one camera is used. This embodiment is particularly useful when light is very ample on one imaging channel, such as a white light camera, and on another imaging channel such as a fluorescence camera, the amount of light received by this camera is very low.

However, the data processing device is not limited to generate only one output image 170 when operating this two cameras. The data processing device 140 may thus also generate and/or output a secondary output image that is calculated from the primary input image sequence by stacking or combining of the images in the sequence.

The data processing device 140 may stack or combine the most recent three, four, five, six, seven, eight, nine, ten, eleven, twelve (or any other number of) images of an input image sequence, in response to determining that no motion is present indeed of the observed object scene.

When the data processing device 140 determines that motion is present in the object scene 120, the data processing device 140 may adjust a frame rate setting of the camera 130 in order to use motion blur artefacts. When the medical observation device 100 is operated with two cameras observing the object scene 120 simultaneously, the data processing device 140 may adjust the frame rate setting of both cameras. In any case, the data processing device may increase the frame rate setting of the primary camera 130 and/or secondary camera 150.

Furthermore, in response to determining that motion is present in the observed object scene 120, the data processing device may determine a stationary image region or a stationary image segment in the input image sequence. Hereinafter the term stationary image region and stationary image segment are used synonymously. The stationary image region may be an (entire) image or a mask of an image and indicates input pixels in the input images of the input image sequence that do not substantially change in the input image sequence. In other words, these input pixels are indicative or represent stationary portions of the recorded object scene 120. This stationary portion may, for example, be a background behind a moving object.

The data processing device 140 may determine the stationary image region by evaluating or analyzing the input image sequence to determine the motion in the input image sequence. For example, when the result of evaluating the input image sequence is a mask of input pixels that have changed throughout the input image sequence, the complementary set to these input pixels represents the stationary image region. Alternatively, the data processing device may directly produce as a result of evaluating the input image sequence the stationary image region. In any case, the stationary image region indicates or represents positions of the input pixels that are not subject to fluctuations in an image of the evaluated or analyzed input image sequence.

This image may, for example, be the most recent image in the input image sequence. The data processing device 140 may generate and/or output the output image 170 by assembling or composing different regions of the output image depending on the position of an output pixel in the output image. When the position of the output pixel lies in the stationary image region, the intensity value of the output pixel is computed from a combination of intensity values from a subset of a predetermined input image sequence. When only one camera is operating, the predetermined input image sequence is the input image sequence 130.

When two cameras are used, alternatively or additionally, the predetermined input image sequence may be the secondary input image sequence that is retrieved from a secondary camera simultaneously observing the object scene 120. In the combination the input pixels of different input images of the subset that match to the output pixel with respect to the position in the stationary image region are combined. The combination may be computed as sigma stacking, averaging, or median filtering. For the sake of completeness, it is noted that when two cameras are used, the data processing device computes the combination of the output pixel by combining secondary input pixels of different secondary input images of the subset that match to the output pixel with respect to the position in the stationary image region.

To summarize, the intensity value of an output pixel of the output image, wherein the output pixel lies within the stationary image region, is computed from corresponding input pixels of different input images from a subset of the input image sequence. The cardinality of the subset may be two to twelve. The data processing device 140 may generate the subset of the input image sequence by filtering or omitting input images from the input image sequence that has low signal-to-noise ratios. This may further increase the image characteristics in the output image 170. The data processing device 140 may compute the dynamic region of the output image 170, that is, the region of output pixels that lie outside of the stationary image region by adopting the respective intensity value of the input pixel in the most recent input image that matches to the respective output pixel in the output image. Thereby, in the stationary image region of the output image, a still background may be output with a high signal-to-noise ratio while, at the same time, dynamic image regions, that is, the region complementary to the stationary image region, of the output image do not suffer from motion blur artefacts. Thus, the overall imaging characteristics of the medical observation device 100 are increased.

When the medical observation device 100 operates with two cameras, the data processing device 140 may alternatively or additionally generate and/or output the output image 170 from a combination of the secondary input image sequence in an analogous manner. This embodiment is particularly useful when a fluorescence camera simultaneously observes the object scene 120 with a white light camera 130. In this case, the white light camera 130 provides the primary input image sequence for the data processing device 140 to evaluate whether a motion is present in the observed object scene 120. If motion is present, the data processing device 140 will use this information to furnish an improved output image 170 from the secondary input image sequence of the fluorescence camera 150. In particular, the data processing device 140 may compute an intensity value of an output pixel that lies within the stationary image region determined from the primary input image sequence of the white light camera 130.

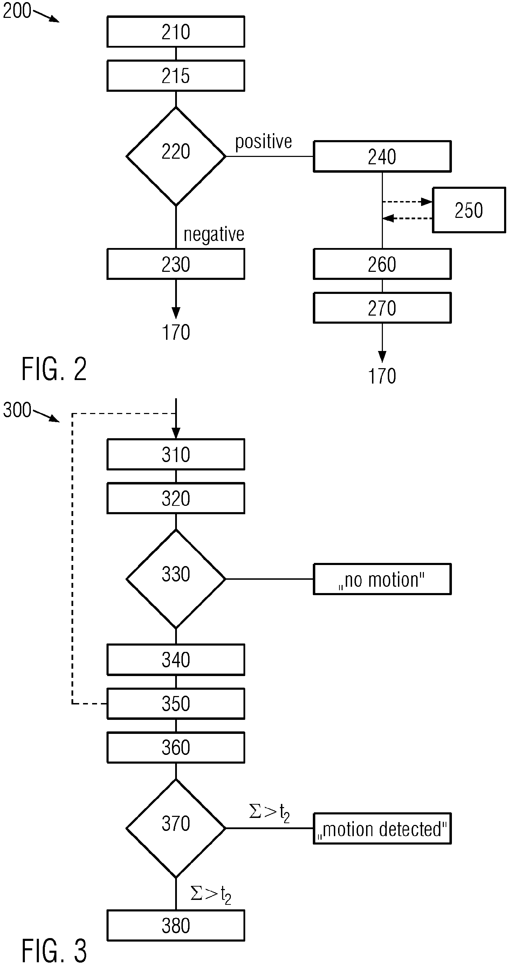

Fig. 2 illustrates a flow diagram of a computer-implemented method for image processing of a medical observation input image that represent an object scene observed by a medical observation device. The data processing device 140 may be configured to implement the computer-implemented method 200. At step 210, an input image sequence of chronologically ordered input images representing the object scene observed by the medical observation device 100 may be retrieved. Additionally, at step 215, in addition to the input image sequence, which may also be referred to as the primary input image sequence, a secondary input image sequence of chronologically ordered secondary input images representing the object scene observed by a second camera of the medical observation device may be received. In this alternative embodiment, the input images of the primary input image sequence are also referred to as primary input images. The input image comprises input pixels and each input pixel comprises an intensity value. Analogously, the input pixels of the primary input images are hereinafter referred to as primary input pixels and, accordingly, the input pixels of the secondary input images are accordingly referred to as secondary input pixels. In any case, each input pixel comprises an intensity value. At step 220, the input image sequence or respectively the primary input image sequence may be evaluated to determine a motion in the observed object scene. If the result of the evaluating of the image sequence is negative, that is, when no motion is determined in the evaluated image sequence 132, an output image 170 may be generated by combining images of the image sequence 132. For example, the output image 170 may be a result of a stacking operation based on the image sequence, or, respectively, the primary input image sequence. For example, in any case, a subset of the input image sequence may be used to generate the output image. In the subset, one or more input images respectively primary input images, may be filtered out from the input image sequence, when a signal-to-noise ratio is below a predetermined threshold value.

At step 230, the output image may be generated by stacking the input image sequence, or respectively the primary input image sequence.

At step 240, a stationary image region may be determined. In particular, the stationary image region may be determined from a result of evaluating the input image sequence or respectively the primary input image sequence.

Optionally, at step 250, a frame rate setting of the medical observation device 100 may dynamically be adjusted. For example, the frame rate setting may be increased. Then, the amount of time that lapses between consecutive images in the input image sequence or respectively primary input image sequence becomes shorter. This allows to reduce motion blur artefacts. The framerate setting may be increased for the primary camera 130 or for both cameras, that is, also for the secondary camera 150, at the same time.

At step 260, an intensity value of an output pixel to be generated for an output image may be computed. The intensity value may be computed from the input image sequence or a subset of the input image sequence, the primary input image sequence or a subset thereof, and/or the secondary input image sequence or a subset thereof. The intensity value of an output pixel that lies in the determined stationary image region may be computed, wherein the intensity value is computed from a combination of intensity values of a subset of a predetermined input image sequence. Predetermined input image sequences may be indicated by an operation setting of the medical observation device 100.

In case the medical observation device is operated with only one camera, the predetermined input image sequence must not be indicated by an operation setting, but is identical to the input image sequence. In case the medical observation device 100 operates with two cameras, the predetermined input image sequence may be the secondary input image sequence, and thus the intensity value of the output pixel in the determined stationary image region is computed from a combination of intensity values of a subset of the secondary input image sequence.

In any case, in the combination of intensity values, the input pixels of different input images of the subset that match to the output pixel with respect to the position in the stationary image region are combined. For example, the primary input pixels of different primary input images of the subset of the predetermined input image sequence that match to the output pixel with respect to the position in the stationary image region are combined. According to another example, in the combination, secondary input pixels of different secondary input images of the subset of the secondary input image sequence that match to the output pixel with respect to the position and the stationary image region are combined.

The position of the output pixel is related to the stationary image region by one or more indices or addresses associated with the output image. Furthermore, an output pixel of the output image that lies outside the stationary image region may be computed from the input image sequence or respectively secondary input image sequence.

The intensity value of an input pixel or respectively of a secondary input pixel may be adopted as the intensity value of the output pixel, wherein the position of the input pixel matches the position of the output pixel in the stationary image region. Alternatively, the intensity value of the output pixel may be computed from a combination of input pixels that match within a sub-region to the output pixel. For example, the intensity value of the output pixel is the average or median of neighboring input pixels of an input pixel that matches to the output pixel with respect to the position and the number of neighboring input pixels in the same input image is defined by a configurable parameter. For example, the configurable parameter for the size of neighboring pixels that should be taken into account to compute the output pixel may be dynamically adjusted in response to an adjustment to the frame rate setting. Thereby, in case the used frame rate setting introduces too much noise components into the recorded images may be compensated.

At step 270 the output image 170 may be output. For example, the output image may be output to the output device 160. The output device 160 may receive the output image 170 from the data processing device 140. Likewise, two separate output images, one for each camera observing the object scene 120 simultaneously may be provided to the output device 160.

Fig. 3 shows a schematic flow diagram of a method for evaluating the input image sequence to determine a motion in the object scene. In the following, the method is described in terms of input pixels. However, the same method applies without any restrictions also to primary input pixels or the secondary input pixels.

As input images, such as the primary input images or the secondary input images, are digital images processed or processable by a computer, a position of any input pixel in an input image may be represented by a pair of numbers or indices. Such a pair of numbers is also known to the skilled person as pixel coordinates. The pair of numbers represents a position of the input pixel in a two-dimensional area.

The method 300 for evaluating a motion in the object scene may be performed by parallel computation or sequential computation. When it is performed by sequential computation, a first input pixel with which the computation starts may be set at step 310.

At step 320 a variance or standard deviation for a given or the already set input pixel is computed. As the variance is defined as the square of the standard deviation, preferably only the absolute value of the standard deviation may be computed. To compute the aforementioned statistical measure the intensity values of input pixels in the input image sequence are used, wherein each input pixel is located at the same position in different input images of the input image sequence. Since the position of the given or set input pixel translates to a location in the object scene, the input image sequence comprising the input images, thus provides a time series of intensity values at the given or set position of the input pixel. At step 330, the computed measure such as the standard deviation, the variance, or the absolute value of the standard deviation, may be compared against a predetermined threshold. The statistical measure may be compared to determine whether it exceeds the predetermined threshold. If the computed statistical measure were low, it may indicate that no motion is present at the given or set input pixel.

At step 340, any input pixels in a given input image, such as the most recent input image in the evaluated input image sequence, may be marked or labelled. For example, the input pixels may be marked as moving pixels. Alternatively, instead of marking the moving pixels, any pixels that do not exceed the predetermined threshold may be marked. In this case they may be marked as stationary pixels.

At step 350, the above steps 310 to 340 may be repeated for all input pixels in case of sequential processing.

At step 360, the sum of marked input pixels may be determined.

At step 370, the sum may be compared against another predetermined threshold t₂ that is indicative of motion in an object scene.

If the sum exceeds the other predetermined threshold t₂, a motion in the object scene may be determined. In other words, the fluctuation of intensity values in subsequent input images indicates that there is motion in the object scene.

Optionally, at step 380, a stationary image region may be determined from a result of marking the input pixels. For example, when the input pixels were marked as moving pixels, a complementary image mask to the marked input pixels may be determined. This mask represents a stationary image region as it indicates the position of those input pixels where no motion is present in the object scene. Alternatively, when the stationary pixels were marked at step 340, this result may directly adopted as the stationary image region.

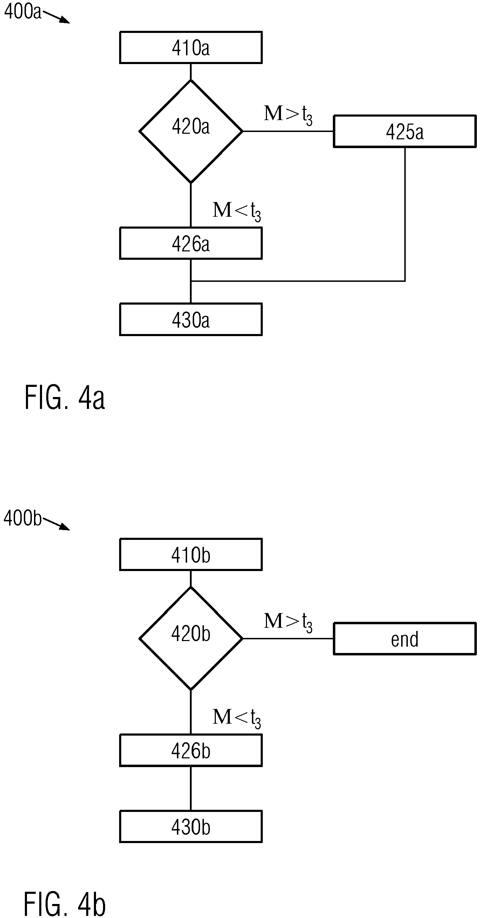

Fig. 4a and 4b illustrate flow diagrams for determining the stationary image region. Fig. 4a shows at step 410a that a statistical measure for each input pixel may be computed pixel-wise. For example, the statistical measure may be the variance and the deviation or the absolute value of the standard deviation. The statistical measure M may be computed along the same input pixel in the input image sequence.

At step 420a, the computed statistical measure M of each input pixel may be compared with a given threshold value t₃. If it is determined that the statistical measure M exceeds the predetermined threshold value t₃, the input pixel may be marked as a moving pixel or as a dynamic object region in step 425a. If it is determined that the statistical measure M is smaller than the predetermined threshold value t₃, the input pixel may be marked as a static pixel or a static object region in step 426a.

At step 430a a complementary mask to the marked pixels may be determined. This complementary mask represents input pixels that are not indicative of moving pixels, or in other words represent stationary input pixels. This mask may be used as the stationary image region.

In Fig. 4b, the statistical measure M is computed in the same manner at step 410b as it was computed in step 410a for Fig. 4a. Likewise in step 420b the computed statistical measure M is compared against the predetermined threshold value t₃. However, in contrast to the method in Fig. 4a, in step only those input pixels are now marked in step 426b, for which the computed statistical measure M falls below the predetermined threshold value t₃. This allows to directly generate a mask that represents the stationary image region in step 430b.

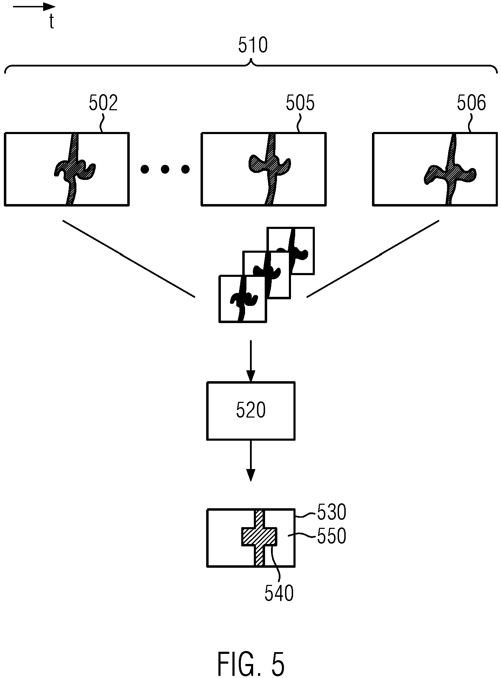

Fig. 5 illustrates a schematic flow chart of a method to determine the stationary image region. On top of Fig. 5 an exemplary input image sequence 510 is illustrated. The input image sequence of chronologically ordered input images 502 to 506 may be processed by a motion detection scheme, a motion estimation scheme, a segmentation analysis scheme or the like at a step 520. Input images 503 and 504 are not shown in the figure.

Step 520 may be part of the previously described methods 300 and 400a and 400b. As the result of step 520, the stationary image region may be obtained. For example it may be obtained from a result of the motion detection scheme, motion estimation scheme or the segmentation analysis scheme. For example, any or all of the aforementioned schemes may produce a mask which segments an input image of the input image sequence into at least two parts, wherein one part or segments represents the stationary image region and another part that represents a dynamic image region.

The dynamic image region may represent moving pixels. On the bottom of Fig. 5 an example mask 530 comprising a dynamic image region 540 (shaded) and a stationary image region 550 is illustrated. In the given example, the stationary image region 550 is non-continuous in that the central input pixels are labelled by the moving pixels of the dynamic image area 540.

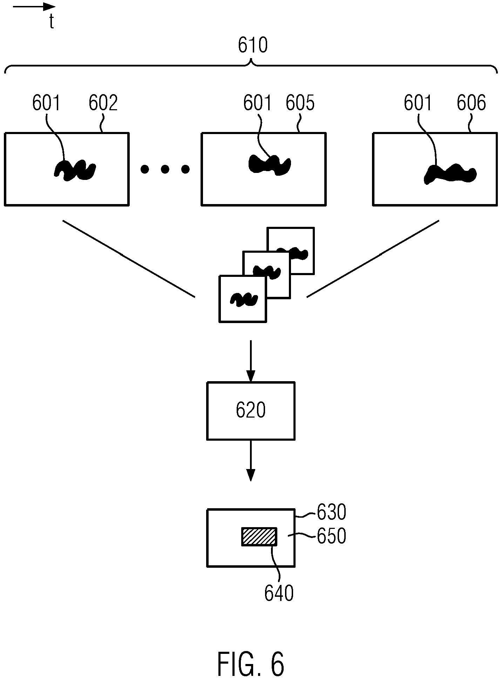

Fig. 6 illustrates another schematic flow chart for determining a stationary image region starting from another image sequence. In the input image sequence the chronologically ordered input images 602 to 606 represent the object scene as observed by a camera of the medical observation device 100.

In the given example, a structure 601 in the center of the images respectively object scenes moves from one direction towards another direction through the center of the images. At step 620 the stationary image region may be determined from analyzing the input image sequence 610. In particular, as a result of the analyzing or evaluating of the input image sequence 610, an image mask 630 may be generated, wherein the mask 630 also comprises pixels and, depending on whether the pixel indicates a motion or not in the object scene, the intensity value of the pixel is set to a value that indicates a motion or does not indicate a motion.

In the illustrated image mask 630, the stationary image region 650 is continuous and at the center it entirely encircles the dynamic image region 640.

## Claims

1. A data processing device (100) for motion-dependent image processing in a medical observation device, such as a microscope or endoscope, the data processing device (100) being configured to:
- retrieve an input set (102a) of input images from a plurality of input images (102), the plurality of input images being part of one or more time sequences of input images,
wherein the input images of the input set (102a) are all from the same time sequence of input images, and
wherein each input image comprises input pixels, each input pixel comprising an intensity value;
- determine a stationary input image segment (104) in the input images of the input set (102a) by applying a motion detection scheme to the input set (102a) , the stationary input image segment (104) representing stationary image content in the input set (102a);
- determine a stationary output image segment (108) in an output image (106) , the stationary output image segment (108) having the same location in the output image (106) as the stationary input image segment (104) has in one of the input images of the input set (102a); and
- determine an intensity value of an output pixel of the output image (106) in the stationary output image segment (108) from a combination of intensity values combining corresponding input pixels from different input images of a subset of input images with each other, wherein the corresponding pixels lie within the stationary input image segment (104).

2. A data processing device (100) according to claim 1, wherein the data processing device (100) is further configured to:
- determine an intensity value of an output pixel of the output image (106) outside (110) from the stationary output image segment (108) from one or more corresponding input pixels of the subset of the input images outside the stationary input image segment; and
a number of input pixels used for determining the intensity value of the output pixel outside (110) the stationary output image segment (108) being smaller than a number of input pixels used for determining the intensity value of an output pixel in the stationary output image segment (108).

3. A data processing device according to claim 2, wherein the data processing device (100) is further configured to:
- determine the intensity value of an output pixel outside (110) the stationary output image segment (108) as the intensity value of a corresponding input pixel of the most recent input image of the subset of the input images.

4. A data processing device (100) according to claim 2 or 3, wherein the number of input pixels used for determining the intensity value of an output pixel in the stationary output image segment (108) is in the range of 3 to 9.

5. A data processing device (100) according to any one of claims 1 to 4, wherein the data processing device (100) is configured to:
- retrieve a second set (102b) of second input images from the plurality of input images,
wherein the second set (102b) of second input images are all from the same time sequence, the time sequence of the second set (102b) of input images being different from the time sequence of the input set (102a), and
wherein each second input image comprises second input pixels, each second input pixel comprising an intensity value;
- determine a second stationary output image segment in each of the second input images of the second set (102b), the second stationary output image segment being in a second output image and having the same location in the second output image as the stationary input image segment (104) has in one of the input images of the input set (102a);
- determine an intensity value of an output pixel in the second stationary output image segment of the second output image from a combination of the intensity values combining corresponding input pixels from different input images of a subset of the second set (102b), wherein the corresponding pixels lie within the stationary input image segment (104).

6. A data processing device (100) according to any of the preceding claims, the data processing device (100) further configured to:
- register the input images of the input set and the second input images of the second set.

7. A data processing device (100) of claim 6, wherein the input images and second input images have been recorded simultaneously and/or synchronously.

8. A data processing device (100) according to any of the preceding claims, wherein the data processing device (100) is further configured to:
- determine a size of the stationary input image segment; and
- determine the intensity value of the output pixel of the output image (106) in the stationary output image segment (108) from the combination, when the determined size of the stationary input image segment exceeds a combination threshold.

9. A data processing device (100) according to any of the preceding claims, the data processing device (100) further configured to:
- determine a standard deviation for the output pixel in the stationary output image segment (108) from the corresponding pixels of the different input image that lie within the stationary input image segment (104).

10. A data processing device (100) according to any of the preceding claims, wherein the data processing device (100) is configured to:
- compute the output image (106), the stationary input image segment (104), or the stationary output image segment (108);
- provide the output image (106) to a display device (112).

11. Medical observation device, such as a microscope, an endoscope, the medical observation device comprising:
- a data processing device (100) according to any one of claims 1 to 10;
- an input camera (114) for recording a time sequence of the one or more time sequences.

12. Medical observation device according to claim 11, wherein the medical observation device further comprises:
- a second camera (116) for recording a second time sequence of the one or more time sequences.

13. Medical observation device according to claim 12,
- wherein the input camera (114) is a white light camera; and/or
- wherein the second camera (116) is a fluorescence light camera configured to record an emission spectrum of at least one fluorophore.

14. A computer-implemented method for motion-dependent image processing of medical ob-servational input images, the computer-implemented method comprising:
- retrieving an input set (102a) of input images from a plurality of input images, the plurality of input images being part of one or more time sequences of input images,
wherein the input images of the input set (102a) are all from the same time sequence of input images, and
wherein each input image comprises input pixels, each input pixel comprising an intensity value;
- determining a stationary input image segment (104) in the input images of the input set (102a) by applying a motion detection scheme to the input set (102a), the stationary input image segment (104) representing stationary image content in the input set (102a);
- determining a stationary output image segment (108) in an output image (106), the stationary output image segment (108) having the same location in the output image as the stationary input image segment (104) has in one of the input images of the input set (102a);
- determine an intensity value of an output pixel in the stationary output image segment (108) of the output image from a combination of intensity values combining corresponding input pixels from different input images of a subset of input images with each other, wherein the corresponding pixels lie within the stationary input image segment (104).

15. A computer-readable medium or a computer program product comprising instructions which, when the program is executed by a computer, cause the computer to carry out the computer-implemented method according to claim 14.
